# EUROPEAN PATENT APPLICATION

(11) **EP 4 541 871 A1**
(43) Date of publication of application: **23.04.2025**
(21) Application number: 24202575.7
(22) Date of filing: 25.09.2024
(51) Int. Cl.: C10G 3/00, C07C 67/08, C07C 69/24, C10L 1/02, C11C 3/00

(54) **BIOFUEL AND PROCESS FOR PREPARATION THEREOF**

(30) Priority: 16.10.2023 US 202363590632 P
(71) Applicant: Galata Chemicals, LLC, Jersey City, NJ 07311 (US)
(72) Inventor: FRENKEL, Peter, Danbury, CT, 06811 (US)
(74) Representative: Prinz & Partner mbB

(57) **Abstract**

A process including either transesterifying seed oils or animal fats with an octanol selected from the group consisting of n-octanol, iso-octanol, 2-ethylhexanol or mixtures thereof in the presence of a catalyst selected from alkali metal n-octoxide, alkali metal iso-octoxide or alkali metal 2-ethylhexoxide, or esterifying fatty acids with an octanol in the presence of a sulfonic acid, thereby forming methanol-free and ethanol-free octyl esters of fatty acids.

## Description

### FIELD OF THE INVENTION

The present invention relates to the field of biofuels that can be used as heating fuel and/or for powering engines to replace hydrocarbon diesel.

### BACKGROUND OF THE INVENTION

Commonly, biodiesel is produced by trans-esterifying seed oils or fats (such as soybean oil, canola oil or tallow) with an excess of anhydrous methanol or ethanol in the presence of catalytic amounts of NaOH, KOH or sodium methoxide. It results in the formation of methyl esters of fatty acids or ethyl esters of fatty acids (biodiesel), respectively, glycerol and recovered excess of the alcohol.

Optionally, the fat and oil triglycerides can be initially split (hydrolyzed) into fatty acids and glycerol, and after separating glycerol from the fatty acids, are esterified with an excess of methanol or ethanol. Methanol is a Hazardous Air Pollutant (Under the Clean Air Act, EPA list of hazardous air pollutants since 1990), and ethanol is also a known highly regulated substance. Both methanol and ethanol are also very flammable raw materials or components (flash point of methanol is 11-12°C and flash point of ethanol is 13°C).

Work has been ongoing for the development of biofuels.

US 8,026,381 describes a continuous process for manufacturing ethyl esters (biodiesel) comprising the products prepared by hydrolyzing triglycerides of vegetable oils to fatty acids and glycerol by passing the fatty acids through CaO/MgO catalysts and esterifying the produced fatty acids with ethanol in a second process step.

WO 2017/187237 describes a process for manufacturing biodiesel from animal tallow comprising the products prepared by transesterification of the tallow with excess methanol in the presence of a sodium methoxide catalyst.

WO 2021/184104 discloses the process for manufacturing esters of fatty acids (biodiesel) by reacting bio-sourced triglycerides with a mixture of methanol and dimethyl carbonate. The obtained biodiesel contained methyl esters of fatty acids, fatty acid glycerol esters, dimethyl carbonates, and traces of methanol. The process is carried under high pressure and temperature.

US 8,124,572 describes a process for producing biodiesel by hydrolyzing triglycerides to fatty acids, separating and esterifying mono-unsaturated fatty acids with methanol to biodiesel and converting saturated and polyunsaturated fatty acids to paraffins for use as lubricants.

U.S. 6,388,800 describes preparation of alkyl fatty acid esters by esterifying saponified feedstock with an alcohol in the presence of an inorganic acid catalyst (such as sulfuric acid), where the alcohol is a straight chain C₁₋₆ alcohol, such as methanol, ethanol or mixtures thereof.

U.S. 6,855,838 describes preparation of alkyl fatty acid esters by esterifying saponified feedstock with an alcohol in the presence of an inorganic acid catalyst, such as sulfuric acid, where the alcohol is a C₁₋₄ alcohol.

Several approaches for manufacturing biofuels were reviewed in "Synthesis of Transportation Fuels from Biomass: Chemistry, Catalysts, and Engineering" by G.W. Huber, S. Iborra, and A. Corma; Chem. Rev. 2006, 106, 9, 4044-4098.

In general, alkyl fatty acid esters can be obtained by either esterifying fatty acids with an alcohol in the presence of an acid catalyst, or by trans-esterifying fatty acid triglycerides with an alcohol in the presence of either acid or alkali catalyst Daniel Swern; Bailey's Industrial Oil and Fat Products, Volume 2, 4th edition, 1982, pp. 113-118 and 130-133*.*

Production and use of methanol-free and ethanol-free fatty acid esters as transportation fuel (biodiesel) and/or heating fuel manufactured from fatty acid feedstocks and octanol, such as 2-ethylhexanol as a main component has not been mentioned in literature.

### SUMMARY OF THE INVENTION

The subject matter of the present disclosure relates to processes for producing biofuels.

In one embodiment, the present disclosure provides a process comprising: transesterifying seed oils or animal fats with an octanol selected from the group consisting of n-octanol, iso-octanol, 2-ethylhexanol and mixtures thereof in the presence of a catalyst selected from alkali metal n-octoxide, alkali-metal iso-octoxide or alkali-metal 2-ethylhexoxide, thereby forming methanol-free and ethanol-free esters of fatty acids. Such materials can be used as biofuels, and not only offer an environmentally safer choice compared to conventional materials but have excellent heat of combustion, are less ignitable and therefore safer, but also have a high flash point.

In another embodiment, the present disclosure provides a process comprising: esterifying fatty acids with an octanol selected from the group consisting of n-octanol, iso-octanol, 2-ethylhexanol or mixtures thereof in the presence of a sulfonic acid catalyst, thereby forming methanol-free and ethanol-free esters of fatty acids.

### DETAILED DESCRIPTION OF THE INVENTION

The subject matter of the present disclosure provides a process to produce biofuel. It has surprisingly been found that methanol-free and ethanol-free esters of fatty acids and 2-ethylhexanol function as a high efficiency biofuel. Such fuels are desirable from an environmental standpoint as well as being safer. Both methanol and ethanol are very flammable and have low flash points of 11-12 and 13 °C, respectively. In contrast, the flash point of 2-ethyhexanol is substantially higher at 81°C.

The 2-ethylhexyl fatty acid esters of the present subject matter were obtained by either esterifying fatty acids with 2-ethylhexanol in the presence of a sulfonic acid catalyst or by transesterifying fatty acid triglycerides with 2-ethylhexanol in the presence of the alkali metal alkoxide catalyst, where the alkoxy groups exclude methoxy and ethoxy groups, and the exemplary alkali metal alkoxide used here was potassium 2-ethylhexanoate. For the purposes of this specification, the term esterification means the reaction between a fatty acid and n-octanol, iso-octanol or 2-ethylhexanol. The term transesterification means the reaction between a triglyceride of fatty acids and n-octanol, iso-octanol or 2-ethylhexanol. The term methanol-free means that the octyl esters contain < 10 ppm methanol. The term ethanol-free means that the octyl esters contain <10 ppm ethanol or are below the detection limit of 10 ppm. In either case, the process is carried out at ambient pressure and within a temperature range of 80-180°C.

The fatty acids can be obtained either directly from renewable feed stocks (such as tall oil fatty acids) or by hydrolyzing the fatty acid triglycerides, such as fats (lard) and seed oils, including soybean oil, palm oil, olive oil, cotton seed oil, linseed oil, safflower oil, sunflower oil, canola oil, rapeseed oil, jatropha oil, algae oil, corn oil, tung oil, castor oil and mixtures thereof Daniel Swern; Bailey's Industrial Oil and Fat Products, Volume 2, 4th edition, 1982, p. 99-104*.* Soybean oil and canola oil are preferred. Soybean oil is the most preferred.

### Octanol

Production of the methanol-free and ethanol-free octyl fatty acid esters via transesterification of fatty acid triglycerides according to the present subject matter is conducted with an octanol selected from n-octanol, iso-octanol or 2-ethylhexanol. Preferably, the octanol is 2-ethylhexanol.

### Catalyst

For production of the present fatty acid esters, a conventional sodium methoxide catalyst cannot be used, since it forms traces of methanol upon hydrolysis with atmospheric moisture, and the methanol trace becomes an impurity of the alkyl fatty acid esters. A suitable alkali metal alkoxide (formed by reacting an alcohol with an alkali metal hydroxide) catalyst of the present subject matter was found to comprise sodium or potassium alkali metals and a RO alkoxide groups, where R is a linear, branched, or cyclic hydrocarbon group, containing from 3 to 10 carbon atoms. Preferably, R is n-propyl, iso-propyl, n-butyl, tertiary-butyl, tertiary-pentyl, iso-octyl and 2-ethylhexyl groups. More preferably, the catalyst is selected from alkali metal n-octoxide, iso-octoxide or 2-ethylhexoxide. Examples of those catalysts are potassium n-octoate, potassium iso-octoate or potassium 2-ethylhexanoate. When transesterification is practiced, the catalyst is preferably potassium 2-ethylhexoxide if the alcohol chosen for trans-esterifying soybean oil was 2-ethylhexanol. It ensures that no additional organic impurities would be present in the fuel. When esterification is practiced, the catalyst is a sulfonic acid. Preferably, the sulfonic acid is p-toluyl sulfonic acid. The catalyst is added either as a pure compound at 5-30% of the amount of fatty acid feedstock or as a solution in a methanol-free and ethanol-free alcohol.

### Biofuel

The biofuel produced in the process of the current subject matter preferably possesses a cloud point of < -10 °C, measured according to ASTM D2500. The Cloud Point of a fuel is the temperature below which a waxy residue forms, giving the fuel a cloudy appearance. This parameter is an important property of the fuel since the presence of solidified waxes can clog filter and injectors in engines. Therefore, cloud point indicates the tendency of the oil to plug filters or small orifices at cold operating temperatures. The biofuel of the invention is a high efficiency material, preferably having a Gross heat of combustion, measured according to (ASTM D240), of >41.87 MJ/kg. The biofuel also preferably has a Kinematic Viscosity @ 40°C, mm²/s, measured according to ASTM D445, of 1.9 to 6.0 mm²/s.

The biofuel produced also preferably has a Pensky-Martens Closed Cup Flash Point of >100°C, measured according to ASTM D93. Flashpoint refers to the temperature at which a flammable liquid vaporizes and can ignite. The lower the flashpoint temperature, the easier it is to ignite the fuel when an ignition source is present. Accordingly, the higher the flashpoint, the safer the liquid is.

The biofuel of the present subject matter is suitable for use as transportation oil or heating oil. For the purposes of this specification, the term transportation fuel means a fuel suitable for consumption by vehicles. These fuels include but are not limited to diesel and the like.

### Examples

The following Examples further detail and explain preparation of the subject biofuels. These examples merely illustrate the invention. Those skilled in the art will recognize many variations that are within the spirit of the invention and scope of the claims.

### Examples of Preparing Biofuels

### Example 1. Preparation of Potassium 2-Ethylhexoxide Catalyst

Charge 160 g of 2-ethylhexanol to a 3-neck 500 ml flask, add 7.5 g a 45% aqueous solution of potassium hydroxide. Place an oval 2.5 cm magnetic stir bar in the flask. Put the flask on a hot plate/magnetic stirrer. Add a takeoff head, (condenser filled with 5° C cooling water), vacuum take off adapter and a 100 ml receiving flask to the middle neck, a thermometer with sealing adapter to a side neck and a valve (closed) to the 3rd neck. Set the 3rd neck up to break vacuum with nitrogen at the end of the catalyst preparation. Place an ice water trap between the vacuum adapter and a vacuum pump. Pull vacuum (30 to 60 mm Hg pressure) and begin agitation and heating. Heat to 80°C and maintain it for 1 hour at 80 to 85° C to remove water.

Increase vacuum to 10 mm Hg pressure at 80 to-85°C for an additional hour to fully dry the batch. Remove heating and cool down the reaction flask to ambient temperature. Break the vacuum with nitrogen and continue the slow nitrogen purge.

### Example 2. Trans-esterification of soybean oil (SBO)

Remove the takeoff head and condenser and replace it with a vented addition funnel in the set up for Example 1. At moderate agitation charge 200 g of dry (<0.1% moisture content according to the Karl Fisher titration method ASTM D6304) soybean via the vented addition funnel placed in the middle neck. Begin heating to 90°C while charging the SBO. When all the SBO is in, remove the addition funnel and close the middle neck with a glass stopper. Make sure it is loose enough to allow the nitrogen purge to exit the flask. Cook at 90 to 125°C with slow agitation under a slight nitrogen purge for 2 hours. Turn off agitation and transfer the reaction content to a separatory funnel. Settle and decant the glycerin (bottom) layer (approx. 18 g) while still hot. Cool down to 65°C.

Transfer the crude product after decanting glycerin to a 600 ml beaker. At 50-60°C, add 120 g slightly acidified water to reach pH 5-6. Settle for 1 hour, decant the aqueous (bottom) layer. Transfer the content to a 500 ml vacuum flask with 2.5 cm stirring bar. Place a stopper and a thermometer in the flask. Place the flask over a hot plate with a stirrer. Begin agitation, applying vacuum and heat. Dry at 60-65°C. under vacuum (30 mm Hg pressure) to remove excess of the alcohol and residual water, achieving moisture content of < 0.1% by the Karl Fisher method.

Apply high vacuum (2 to 3 mm Hg pressure) until the distillation rate slows to 1 drop per 5 - 10 seconds (alcohol content should be 3% or less). Cool down the product to 60°C and break the vacuum.

### Example 3. Esterification of soybean oil fatty acids with 2-ethylhexanol

Charge 220 kg of soybean oil fatty acids, 150 kg 2-ethylhexanol and 0.75 kg p-toluyl sulfonic acid into a reactor. Under agitation, heat the reaction mixture up to 104°C. Maintain reaction temperature of 104 °C under agitation, a nitrogen blanket and full vacuum for 6 hours. Turn off the agitation, cool down the reaction content, settle the two phases, separate the aqueous (bottom) layer, turn off nitrogen, wash the product layer with 50 kg water, separate the aqueous phase, apply full vacuum to remove residual moisture and obtain the dry product containing < 5% residual 2-ethylhexanol. Conversion of soybean oil fatty acids: 95-99%.

Selected requirements for biofuels and characteristics of 2-ethylhexyl esters of soybean oil fatty acids prepared according to Example 2 and 3 are shown in Table 1 and Table 2, respectively.

**Table 1. Selected requirements for biofuels**

| **Characteristics** | **UK S.20 Specification** |
|---|---|
| Gross Heat of Combustion (MJ/kg) | > 41.87 |
| Kinematic Viscosity @ 40°C, mm²/s | < 125 |
| Pensky-Martens Closed Cup Flash Point, °C | >66 |
| Cloud Point, °C | -3 - +8 |

**Table 2. Selected properties of the obtained biofuel**

| **Property** | **Results** |
|---|---|
| Gross heat of combustion (ASTM D240), MJ/kg | 42.03 |
| Kinematic Viscosity @ 40°C, mm²/s | 5.6 |
| Pensky-Martens Closed Cup Flash Point, °C | 131-250 |
| Cloud Point, °C | -20 |

As it can be seen, methanol-free, ethanol-free 2-ethylhexyl soyate exceeded the minimum requirement for Gross Heat of Combustion and was found to be substantially less ignitable and therefore safer (compare Flash point requirements of >66°C for biodiesel in Table 1 with the Flash Point of 2-ethylhexyl soyate as being >131°C in Table 2). Additionally, 2-ethylhexyl soyate of this invention exhibited considerably lower Cloud Point than it is required (compare the corresponding values in Table 1 and Table 2) that is advantageous, especially if the fuel is used in cold climate areas.

## Claims

1. A process comprising:
- transesterifying seed oils or animal fats with an octanol selected from the group consisting of n-octanol, iso-octanol, 2-ethylhexanol and mixtures thereof in the presence of a catalyst selected from alkali metal n-octoxide, alkali metal iso-octoxide or alkali metal 2-ethylhexoxide, thereby forming methanol-free and ethanol-free esters of fatty acids.

2. The process of claim 1, where the octanol is 2-ethylhexanol.

3. The process of claim 1 or claim 2, wherein the seed oils are selected from soybean oil, palm oil, olive oil, cotton seed oil, linseed oil, safflower oil, sunflower oil, canola oil, rapeseed oil, jatropha oil, algae oil, corn oil, tung oil, castor oil or mixtures thereof.

4. The process of claim 3, wherein the seed oils are soybean oil, canola oil or mixtures thereof.

5. The process of any one of claims 1 to 4, wherein the alkali metal n-octoxide catalyst is potassium 2-ethylhexoxide.

6. A biofuel comprising methanol-free and ethanol-free n-octyl esters of fatty acids, iso-octyl esters of fatty acids, 2-ethylhexyl esters of fatty acids or mixtures thereof prepared according to any one of claims 1-5.

7. A process comprising:
- esterifying fatty acids with an octanol selected from the group consisting of n-octanol, iso-octanol, 2-ethylhexanol or mixtures thereof in the presence of a sulfonic acid catalyst, thereby forming methanol-free and ethanol-free esters of fatty acids.

8. A biofuel comprising the methanol-free and ethanol-free esters produced by the process of claim 7.

9. The biofuel comprising methanol-free and ethanol-free octyl esters of fatty acids of claim 8, where the octanol is 2-ethylhexanol.

10. The biofuel comprising methanol-free and ethanol-free octyl esters of fatty acids of claim 7 and 8, where the sulfonic acid catalyst is p-toluyl sulfonic acid.

11. The biofuel of claims 6, and 8-10 having a Pensky-Martens Closed Cup Flash Point of >100°C.

12. The biofuel of claims 6 and 8-11 having a cloud point of < -10 °C.

13. The biofuel of claims 6 and 8-12 suitable for use as transportation oil or heating oil.

14. A process comprising preparing a transportation oil comprising the methanol-free and ethanol-free esters of fatty acids of any one of claims 1 to 5.

15. A process comprising preparing a heating oil comprising the methanol-free and ethanol-free esters of fatty acids of any one of claims 1 to 5.
